# EUROPEAN PATENT SPECIFICATION

(11) **EP 2 211 952 B1**
(45) Date of publication and mention of the grant of the patent: **22.01.2014**
(21) Application number: 08837613.2
(22) Date of filing: 23.09.2008
(51) Int. Cl.: A61M 5/50, A61M 5/32

(54) **SAFETY NEEDLE**
SICHERHEITSNADEL
AIGUILLE DE SÉCURITÉ

(30) Priority: 11.10.2007 GB 0719876
(43) Date of publication of application: 04.08.2010
(73) Proprietor: Salvus Technology Limited, Stradbroke Suffolk IP21 5HS (GB)
(72) Inventor: WESTON, Terence, Edward, Norfolk NR9 5NP (GB)
(74) Representative: Lippert, Stachow & Partner
(86) International application number: PCT/US2008/077352
(87) International publication number: WO 2009/048737

(56) References cited:
- EP-A- 1 558 311

## Description

### BACKGROUND OF THE INVENTION

This invention relates to a safety needle, particularly a safety needle for automatically covering a needle tip following removal of the needle from a patient.

Needle stick injuries carry a significant risk of spreading serious diseases such as hepatitis and HIV. The risk of spreading an infectious disease is greater immediately after the hypodermic needle is withdrawn from the patient. A number of device are known for providing a sleeve or other shielding element to cover the sharp needle tip after administering the injection. The preferred devices are those devices that are passive - i.e., shields that automatically cover the sharp needle tip after the injection without the user having to perform a special operation to render the needle safe. It is further preferred that the device locks the shield over the needle after use.

A passive safety needle device is disclosed in European Patent No. 1 558 311 (the '311 patent), and the below-described invention is based broadly on that invention. In the '311 patent, the fundamental operating principle is a slidable sleeve having at least one cantilever arm sliding along, and expanding over a conical surface upon insertion of the needle into the patient. On removal of the displacing force caused by the skin of the patient on the slidable sleeve, the stored energy in the cantilever arm causes the sleeve to slide back down the conical surface and become locked in a position so as to cover the sharp needle tip. Practical embodiments employ four cantilever arms rigidly attached to a bushing, the entire slidable sleeve being molded of a resilient polymeric material having the optimum blend of mechanical properties such as high modulus of elasticity and low friction.

A basic requirement of any such device using polymeric springs is that there must be sufficient stored energy in the spring(s) to ensure reliable return of the slidable sleeve to the locked position, and this requires the spring to be at least partially pre-loaded. While the devices described in the '311 patent are very effective and cost-efficient, the device must be stored in an unstressed condition or unloaded position, because the polymeric material will creep over time and loose some of its potential as a spring.

What is needed is a safety needle for automatically covering a needle tip following removal of the needle from the patient that resists creep in the slidable sleeve when kept or stored in a pre-loaded position.

### BRIEF SUMMARY OF THE INVENTION

Briefly stated, the present invention is directed to a safety needle for automatically covering a needle tip following removal of the needle from a patient. The safety needle comprises a hollow needle that has a longitudinal axis and a tip for injecting into the patient. A hub is mounted to the needle and has an outer surface, a receiving end which is distal to the tip of the needle and an injection end which is proximal to the tip of the needle. A slidable sleeve has a mounting end which is distal to the tip of the needle and an injection end which is proximal to the tip of the needle. The mounting end is slidably mounted to the hub between the receiving and injection ends of the hub. The slidable sleeve has an extended position in which the tip of the needle is located inside the slidable sleeve and a retracted position in which the tip of the needle projects from the slidable sleeve. The outer surface of the hub deflects the slidable sleeve in a radial direction as the slidable sleeve slides in an axial direction toward the receiving end of the hub. A spring member is mounted to the slidable sleeve proximate the mounting end. A displacement force urges the slidable sleeve toward the retracted position generating a restoring force within the spring member. The restoring force urges the slidable sleeve to move axially toward the injection end of the needle hub and into the extended position upon removal of the displacement force.

### BRIEF DESCRIPTION OF THE SEVERAL VIEWS OF THE DRAWINGS

The foregoing summary, as well as the following detailed description of preferred embodiments of the invention, will be better understood when read in conjunction with the appended drawings. For the purpose of illustrating the invention, there are shown in the drawings embodiments which are presently preferred. It should be understood, however, that the invention is not limited to the precise arrangements and instrumentalities shown.

In the drawings:
Fig. 1 is a side elevational view of a safety needle in accordance with a first preferred embodiment of the present invention shown in an intermediate or ready-to-use configuration;
Fig. 1a is a cross sectional view of the safety needle of Fig. 1 taken about line A-B of Fig. 1 and having an elastomeric spring member;
Fig. 1b is a rear elevational view of a proximal end of the safety needle shown in Fig. 1;
Fig. 1c is a cross sectional view of the safety needle of Fig. 1 taken about line A-B of Fig. 1 and having a coil spring member;
Fig. Id is an enlarged partial perspective view of a foot in a retaining slot of the safety needle shown in Fig. 1;
Fig. 2 is a side elevational view of a safety needle in accordance with a second preferred embodiment of the present invention shown in an intermediate or ready-to-use configuration;
Fig. 2a is a side elevational view of the safety needle of Fig. 2 partially cut-away to show the internal construction;
Fig. 3 is a cross sectional view of the safety needle of Fig. 2 shown in the retracted position with the needle being injected into a patient;
Fig. 4 is a side elevational view of the safety needle of Fig. 2 partially cut-away and shown in the extended and locked position following an injection;
Fig. a is a rear elevational view of a proximal end of the safety needle shown in Fig. 4;
Fig. 4b is an enlarged partial cross sectional view of a locking member of the safety needle of Fig. 2 in the extended and locked position;
Fig. 5 is a side partial cross sectional elevational view of the safety needle of Fig. 1 shown mounted to a syringe; and
Fig. 6 is a side partial cross sectional elevational view of the safety needle of Fig. 1 shown mounted to a vial syringe.

### DETAILED DESCRIPTION OF THE INVENTION

Certain terminology is used in the following description for convenience only and is not limiting. The words "right", "left", "lower" and "upper" designate directions in the drawings to which reference is made. The words "inwardly" and "outwardly" refer to directions toward and away from, respectively, the geometric center of a safety needle in accordance with the present invention, and designated parts thereof. Unless specifically set forth herein, the terms "a", "an" and "the" are not limited to one element but instead should be read as meaning "at least one". The terminology includes the words noted above, derivatives thereof and words of similar import.

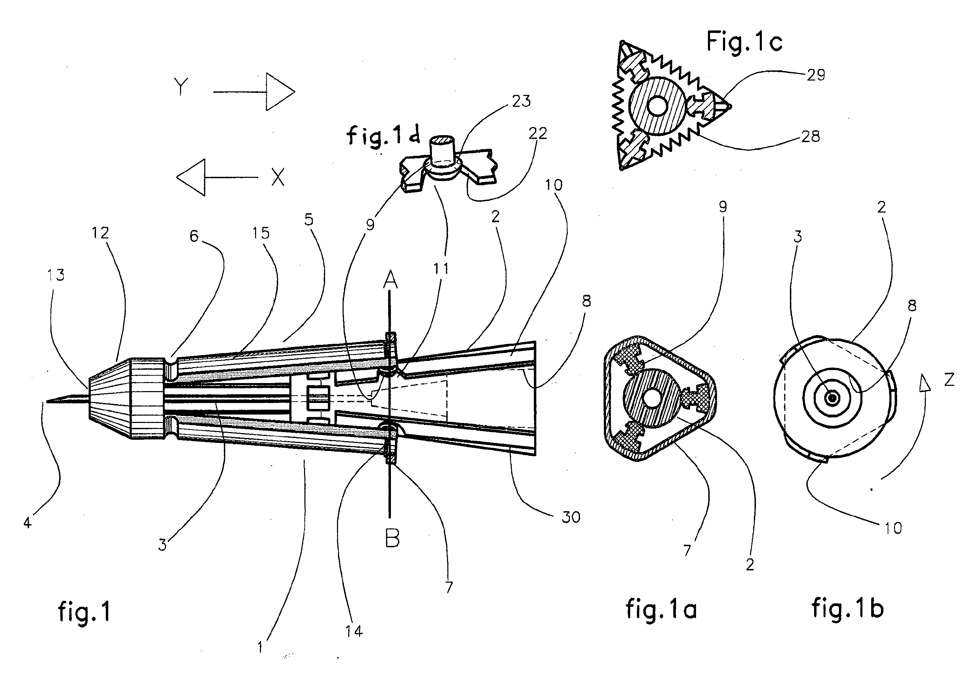

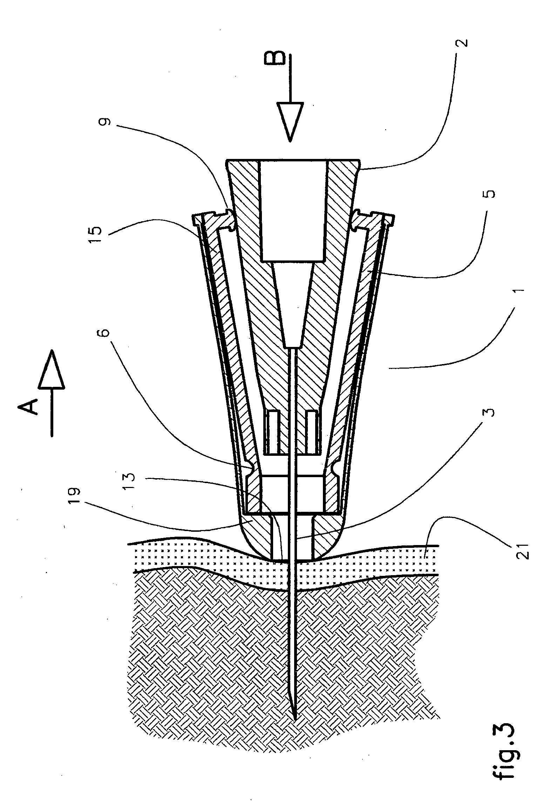

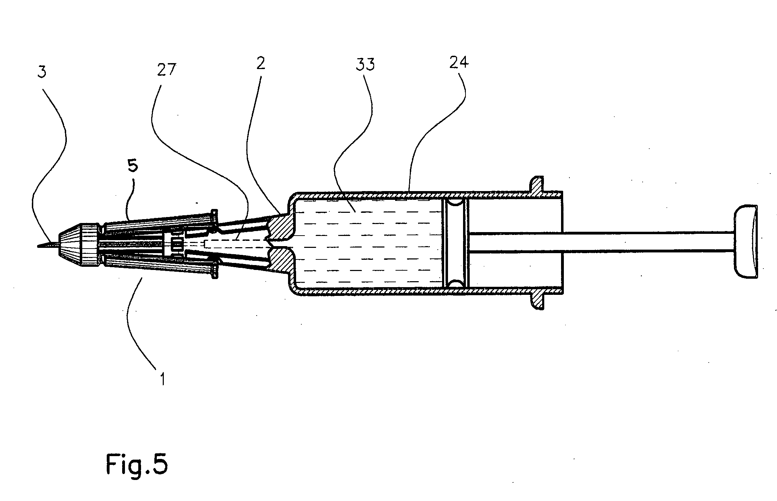

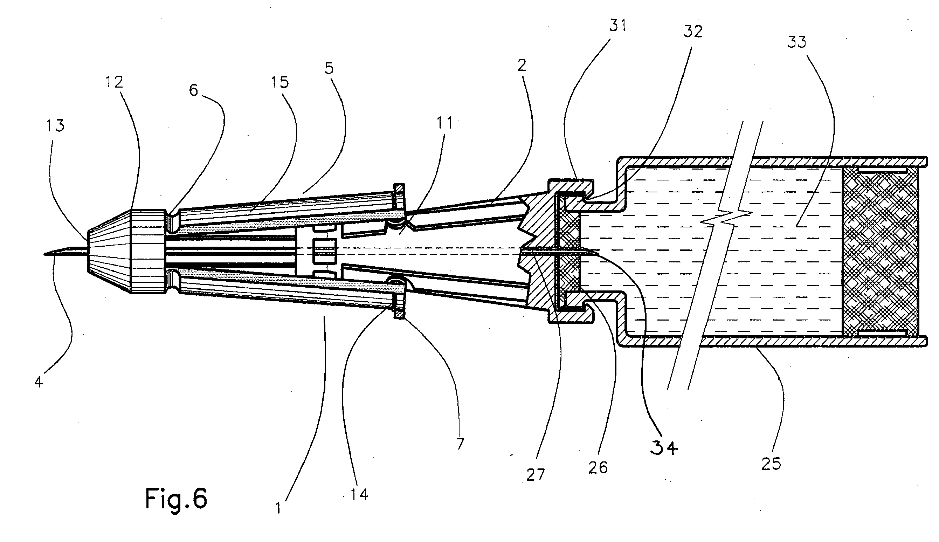

Referring to the drawings in detail, wherein like reference numerals indicate like elements throughout, there is shown in Figs. 1-6, first and second preferred embodiments of a safety needle, generally designated 1, in accordance with the present invention. The safety needle 1 is for automatically, or passively, covering a needle 3, and particularly the tip 4 of the needle 3, following removal of the needle 3 from the skin 21 of a patient (Fig. 3). The needle 3 is preferably fixedly attached to the safety needle 1 but the needle 3 may be separately provided and attached to a syringe 24 (Fig. 5), a dental cartridge 25 (Fig. 6) a vial or vial syringe (not shown) or other delivery device. The safety needle 1 is generally cylindrical but may have any suitable shape. The width of the safety needle 1 is preferably similar to the width of the delivery device with which the safety needle 1 is used.

Referring to Figs. 1-4b, the needle 3 includes a beveled or sharpened tip 4 for injection into the patient and extends co-axially along a longitudinal axis (not shown). A hub 2 is securely mounted to the needle 3 and preferably has a generally conical outer surface that tapers inwardly toward the tip 4. The hub 2 is preferably frusto-conical but may be a pyramid or wedge-shaped with just two converging faces. Also, the surface of the tapered hub 2 need not be linear and changing the slope of the hub 2 may be desirable. The hub 2 has a receiving end distal to the tip 4 of the needle 3 and an injection end proximal to the tip 4 of the needle 3. The receiving end preferably includes a connection mount 8 such as threads or a snap fit for connection to the syringe 24 or other delivery device. The connection mount 8 may also be a Luer Taper, as shown, or Luer Lock (not shown), or any other means to suit the attachment of a syringe 24 or other device. The hub 2 may also be integral with the syringe body 24, which may be pre-filled with a medicament 33 (Fig. 5). The hub 2 may further be adapted to have a cap 31, which is snapped onto the retaining flange 32 of a cartridge 25 (Fig. 6). The hub 2 may have a double ended needle 27 in place of the needle 3, and the action of snapping the safety needle 1 onto the cartridge 25 causes the needle tip 34 to pierce a rubber stopper 26 of the cartridge 25 and make fluid connection with the medicament 33.

A slidable sleeve 5 has a mounting end distal to the tip 4 of the needle 3 and an injection end 13 proximal to the tip 4 of the needle 3. The slidable sleeve 5 preferably comprises at least one and preferably three cantilever arms 15 attached to a bushing 12 by hinges 6. The bushing 12 is proximate the injection end 13, which is placed on the injection site of the skin 21 during use. The cantilever arms 15 are preferably freely pivotably attached to the bushing 12 but may be fixedly mounted to the bushing 12 or each other. The mounting end of the slidable sleeve 5 is slidably mounted to the hub 2 between the receiving and injecting ends of the hub 2. The mounting end of the slidable sleeve 5 preferably includes a plurality of radially inwardly projecting projections or feet 9, one on each cantilever arm 15. The feet 9 are in sliding contact with the outer surface of the hub 2.

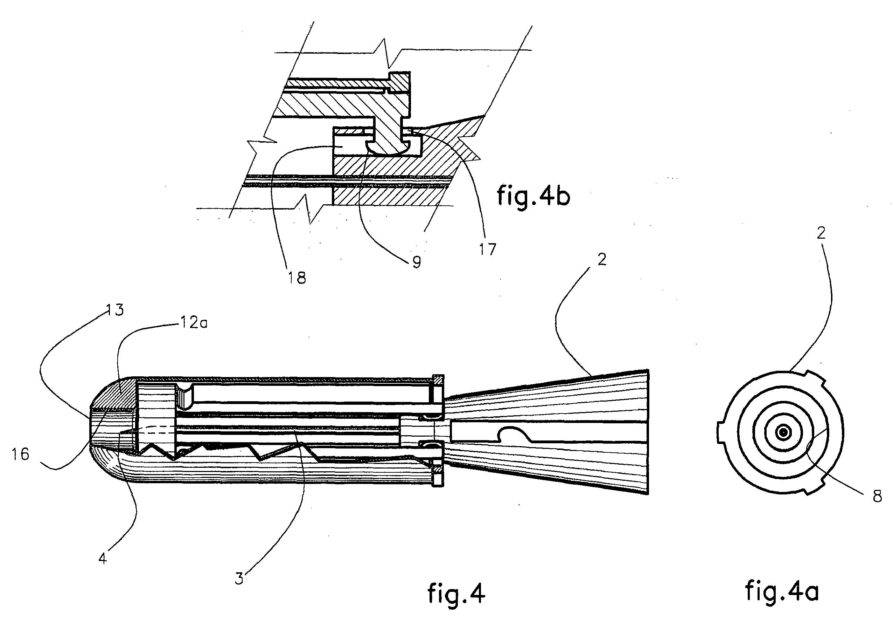

The tip 4 of the needle 3 is located inside of the slidable sleeve 5 in an extended position (Fig. 4) and the tip 4 of the needle 3 projects from the slidable sleeve 5 in a retracted position (Fig. 3). The outer surface of the hub 2 deflects the slidable sleeve 5 in a radial direction as the slidable sleeve 5 is slid along the surface of the hub 2toward the receiving end of the hub 2.

A pre-loaded spring member 7 preferably in the form of a circumambient annular ring is mounted to the slidable sleeve 5 generally proximate the mounting end of the slidable sleeve 5 and radially outwardly from the feet 9. A displacement force in the axial direction Y, caused by the skin 21 abutting the slidable sleeve 5 as the needle 3 is inserted into the skin 21, urges the slidable sleve 5 along the surface of the hub 2 toward the retracted position (see Fig. 3). Such a movement expands the spring member 7 and generates a restoring force within the spring member 7. The restoring force urges the slidable sleeve 5 to move along the outer surface of the hub 2 toward the injection end of the hub in the axial direction X and into the extended position upon removal of the displacement force or upon removal of the needle 3 from the skin 21 (see Fig. 4). Preferably, the spring member 7 expands as it slides up the hub 2 and thus stores energy in the form of a restoring force. On removal of the displacing force in the axial direction Y, the spring member 7 gives up its stored energy to urge the assembly longitudinally along the outer surface of the tapered hub 2 toward the smaller diameter of the hub 2 in the axial direction X.

The spring member 7 may be a garter spring, which comprises one or more expansion springs 28 having the free ends joined on radially extending posts 29 to make a torus or circumambient spring member 7 that extends outer the periphery of the slidable sleeve 5 (Fig. 1c). The expansion springs 28 are preferably constructed of metal but may be constructed of any suitable resilient material. Alternatively, the spring member 7 may be an elastomeric ring (Fig. 1 a). The spring member 7 is preferably constructed of a thermoset or thermoplastic natural or synthetic rubber but may be constructed of any suitable resilient material that has low creep, resistance to aging, resistance to sterilization and low hysteresis. While metallic materials have virtually zero creep in this type of application where the load on the spring member 7 is low, it is preferred that an elastomeric material be used. Elastomers have a creep of less that 0.2% p.a. Thus, the spring member 7 may have a preload which is sustainable over a very long storage life of the safety syringe 1. Any resulting creep of the cantilevers arms 15 is irrelevant with the additional of the spring member 7.

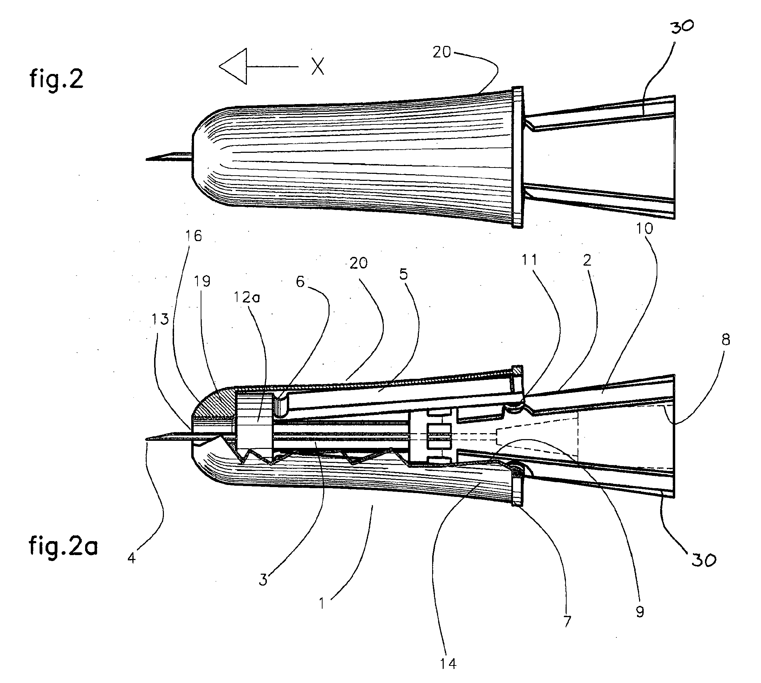

In a first embodiment shown in Figs. 1-1b and 5-6, the spring member is a circumambient ring. In the second embodiment shown in Figs. 2-4b, the arrangement is similar to the first embodiment, but the spring member 7 is integral with or assembled to a sheath 20 which covers the cantilever arms 15 and extends to the needle tip 4.

In Figs. 1 and 2, the safety needle 1 is shown in its ready-to-inject configuration or an intermediate position. In the ready-to-use arrangements shown, the spring member 7 is preferably partially loaded or contains a stored restoring force such that only a small movement of the slidable sleeve 5 is needed to move the slidable sleeve 5 from the ready-to-use arrangement and then rapidly extend over the needle 3 and into the extended position. In the ready-to-use arrangements shown, the slidable sleeve 5 is initially prevented from sliding to the extended position by the engagement of the feet 9 in the retaining slots 11. The retaining slots 11 have a cam surface 22 (see Fig. 1 d) or helical track. Once the slidable sleeve 5 is displaced in the axial direction Y, the feet 9 will be brought into contact with the cam surface 22 and twist the slidable sleeve 5 relative to the hub 2. There may be a small ridge (not shown) in line with an edge 30 of a guide 10 across the entrance to at least one of the retaining slots 11, so that when the displacing force is removed, the feet 9 do not return to rest in the retaining slots 11. However, the ridge could give a resistive feel to the user. Alternatively and preferably, the cross-section of the hub 2 is not truly circular, and is preferably slightly flattened between the guides 10. Such a configuration will cause the slidable sleeve 5 to twist more toward the center line between the guides 10, and avoid returning to the retaining slots 11.

Figs. 2 and 2a show a similar arrangement to that already described, but the spring member is an elastomer sheath 20 having a nose 19. The slidable sleeve 5 preferably has a bushing 12a, and the sheath 20 is assembled over the slidable sleeve 5 to enclose the bushing 12a and cantilever arms 15. In such an arrangement, the elastomer ring 7 may be made integral with the sheath 20. The second embodiment helps to protect the needle and cantilever arms 15 from contamination and tampering. In all other respects the sheath 20 functions similar to the circumambient spring as described in the first embodiment. Alternatively, the spring member 7 and the nose 19 may be attached to one another by elastomer ligaments, located if required to cover the gaps between the cantilever arms 15. The sheath 20 may be molded integrally with the nose 19, the ring 7 and/or the bushing 12a. A further variation is to interspace the spring member 7 with the cantilever arms 15, to form an enclosing slidable sleeve 5 to increase the protection of the needle 3.

Fig. 3 shows the second embodiment of the safety needle 1 in the retracted position. During use, the face 13 of the nose section 19 is pressed against the epidermis 21 of the patient by pushing the hub 2 in the direction of the arrow B. As the needle tip 3 enters the skin 21 and the slidable sleeve 5 abuts the skin 21, further displacement of the needle 3 and the hub 2 displaces the slidable sleeve 5 up the outer surface of the hub 2. The slidable sleeve 5 twists out of the ready-to-use configuration and the restoring force begins to build in the spring member 7 as the slidable sleeve 5 moves toward the retracted position and the spring member 7 is expanded. On withdrawal of the needle 3 from the skin 21, the displacement force is removed and the biasing or restoring force of the spring member 7 created by the interaction of the cantilever arms 15 on the hub 2 returns the slidable sleeve 5 toward the injection end of the hub 2. At the end of the return, the slidable sleeve 5 is in the extended position (Fig. 4), where it prevents access to the needle tip 4. Preferably, a locking mechanism retains the slidable sleeve 5 in the extended position such that the needle 3 cannot be re-used. Preferably, the locking mechanism includes slots 17 in a recess 18 within the outer surface of the hub 2 (Fig. 4b). In the extended position, the feet 9 of the cantilever arms 15, preferably having a mushroom shape, drop through the slots 17 into the recess 18. Any further attempt to move the slidable sleeve 5 axially is prevented by the locking action as the enlarged tips of the feet 9 become trapped under the edges of the slots 17. Any suitable locking device may be used but the advantage of slots 17 as a locking mechanism is that no extra force is necessary to displace pawls, gates or other detent/locking means commonly seen in prior art devices and the spring member 7 may further retain the feet 9 in the slots 17. Such a locking and operating configuration is preferred because the operating force of the slidable sleeve 5 should be low - on the order of less than 3.5 ounce-force, and sudden small variations in operating characteristics can confuse the user into thinking that the device has locked, or perhaps not worked correctly and/or impact the use of the needle 3. The pre-load of the spring member 7 preferably ensures that the feet 9 drop reliably through the locking slot 17 automatically once the needle 3 has been withdrawn from the skin 21 and if the spring member 7 is sufficiently pre-loaded, the spring member 7 retains the feet 9 in the slots 17.

An aspect of safety needles that causes some dispute is whether or not the needle tip 4 should be exposed prior to use (i.e. in the ready-to-use configuration). The advantages of an exposed needle 3 are ease of aspirating trapped air and excess medicament prior to use, and ease of targeting the needle tip 4 on to the injection site. In safety needles where the needle tip 4 is not exposed, aspiration is difficult and liquid medicament usually runs into the safety operating mechanism. However, some people, (e.g. those suffering from "needle phobia", and children) prefer that the needle 3 is initially hidden, and a number of so-called pen injectors for self-injection use such safety needles. Although the present specification describes a safety shielding device wherein the needle 3 is initially exposed immediately before use, the tip 4 may also be hidden or covered before use by extending the length of the slidable sleeve 5. In all of the foregoing descriptions, it has been assumed that the needle tip 4 projects from the face of the nose or bushing 12, in accordance with the current preference of professional healthcare workers. The needle tip 4 may be located inside the nose or bushing 12 and not project from the slidable sleeve 5 in the ready-to-use configuration. This has the aforementioned drawbacks of poor targeting of the needle tip 4 on the injection site, and increased difficulty in aspirating trapped air and medicament. Nevertheless, there are applications where it is preferred that the needle tip 4 is hidden at the start of the injection.

Further, the safety needle 1 may also have an initial position where the slidable sleeve 5 is mounted on the hub 2 between the extended and intermediate positions such that the pre-load on the spring member 7 is reduced. The safety needle 1 can then be armed or set into the intermediate position just prior to use thereby increasing the restorative force in the spring member 7.

Preferably, the safety needle 1 is constructed of materials capable of withstanding at least one of the preferred sterilization methods used for medical devices, for example, gamma radiation, autoclaving, gassing with ethylene oxide, and e-beam.

It will be appreciated by those skilled in the art that changes could be made to the embodiments described above without departing from the broad inventive concept thereof. It is understood, therefore, that this invention is not limited to the particular embodiments disclosed, but it is intended to cover modifications within the scope of the present invention as defined by the appended claims.

## Claims

1. A safety needle (1) for automatically covering a needle (3) following removal of the needle (3) from a patient, the safety needle (1) comprising a hollow needle (3) having a longitudinal axis and a tip (4) for injecting into the patient; a hub (2) mounted to the needle (3) and having an outer surface, a receiving end distal to the tip (4) of the needle (3) and an injection end proximal to the tip (4) of the needle (3); a slidable sleeve (5) having a mounting end distal to the tip (4) of the needle (3) and an injection end (13) proximal to the tip (4) of the needle (3), the mounting end of slidable sleeve (5) being slidably mounted to the hub (2) between the receiving and injection ends of the hub (2), the tip (4) of the needle (3) being located inside the slidable sleeve (5) in an extended position, the tip (4) of the needle (3) projecting from the slidable sleeve (5) in a retracted position, the outer surface of the hub (2) deflecting the slidable sleeve (5) in a radial direction as the slidable sleeve (5) slides in an axial direction toward the receiving end of the hub (2), **characterized in that**
a spring member (7) is mounted to the slidable sleeve (5) proximate the mounting end, a displacement force urging the slidable sleeve (5) toward the retracted position generating a restoring force within the spring member (7) is provided, the restoring force urging the slidable sleeve (5) to move axially toward the injection end of the hub (2) and into the extended position upon removal of the displacement force.

2. The safety needle (1) of claim 1, **characterized in that** the slidable sleeve (5) includes at least one longitudinally extending cantilever arm (15).

3. The safety needle (1) of claim 2, **characterized in that** the slidable sleeve (5) includes 2 to 6 cantilever arms (15).

4. The safety needle (1) of claim 2, **characterized in that** at least one cantilever arm (15) is freely hinged and attached at one end to a bushing (12; 12a) proximate to an injection end (13) of the slidable sleeve (5).

5. The safety needle (1) of claim 1, **characterized in that** the slidable sleeve (5) is releasably retained in an intermediate position between the extended position and the retracted position, urging the slidable sleeve (5) in the axial direction toward the retracted position and releases the slidable sleeve from the intermediate position.

6. The safety needle (1) of claim 5, **characterized in that** the slidable sleeve (5) has at least one radially extending projection and the hub (2) has at least one slanted groove, the at least one radially extending projection being retained in the at least one slanted groove in the intermediate position.

7. The safety needle (1) of claim 1, **characterized in that** at least part of the outer surface of the hub (2) tapers radially inwardly toward the injection end such that, in use, as the needle (3) is inserted into a patient, the slidable sleeve (5) is displaced radially outwardly by the tapered outer surface of the hub (2) as the slidable sleeve (5) moves axially thereby generating the restoring force in the spring member (7).

8. The safety needle (1) of claim 7, **characterized in that** the outer surface of the hub (2) is at least partially conical in shape.

9. The safety needle (1) of claim 1, **characterized in that** a fluid delivery device is provided and the receiving end of the hub (2) is mounted to the fluid delivery device.

10. The safety needle (1) of claim 9, **characterized in that** the fluid delivery device is a syringe (24).

11. The safety needle (1) of claim 1, **characterized in that** the injection end of the hub (2) has a locking mechanism and the slidable sleeve (5) is retained in the extended position by the locking mechanism following removal of the displacement force.

12. The safety needle (1) of claim 11, **characterized in that** the slidable sleeve (5) includes at least one radially inwardly extending projection and the locking mechanism is an indentation in the projection of the hub (2) on the free end of the beam which receives the at least one projection in the extended position.

13. The safety needle (1) of claim 1, **characterized in that** the spring member (7) is a circumambient spring.

14. The safety needle (1) of claim 1, **characterized in that** the spring member (7) is constructed of an elastomeric material.

15. The safety needle (1) of claim 1, **characterized in that** the spring member (7) is constructed of a metallic material.

16. The safety needle (1) of claim 1, **characterized in that** the spring member (7) is a sheath that substantially covers the slidable sleeve (5).

17. The safety needle (1) of claim 1, **characterized in that** the slidable sleeve (5) has at least one cantilever arm (15) which engages a helical track in the outer surface of the hub such that, in use, as the needle is inserted into a patient and the slidable sleeve (5) abuts against the patient, the at least one cantilever arm (15) is displaced circumferentially by the helical track.

18. The safety needle (1) of claim 1, **characterized in that** the slidable sleeve (5) has a first extended position where the slidable sleeve (5) is able to be moved toward the receiving end of the hub (2) and into the retracted position and a second extended position where the slidable sleeve (5) is in a locked position.

## Patentansprüche

1. Sicherheitsnadel (1) zum automatischen Verdecken einer Nadel (3) nach dem Entfernen der Nadel (3) aus einem Patienten, wobei die Sicherheitsnadel (1) eine Hohlnadel (3) mit einer Längsachse und einer Spitze (4) für die Injektion in einen Patienten umfasst; eine an der Nadel (3) montierte Nabe (2) mit einer Außenfläche, einem zur Spitze (4) der Nadel (3) distalen Aufnahmeende und einem zur Spitze (4) der Nadel (3) proximalen Injektionsende; und eine Gleithülse (5) mit einem zur Spitze (4) der Nadel (3) distalen Befestigungsende und einem zur Spitze (4) der Nadel (3) proximalen Injektionsende, wobei das Befestigungsende der Gleithülse (5) zwischen den Injektionsenden der Nabe (2) verschiebbar an der Nabe (2) befestigt ist, wobei sich die Spitze (4) der Nadel (3) in der Gleithülse (5) befindet, wenn diese sich in der ausgefahrenen Position befindet, und wobei die Spitze (4) der Nadel (3) aus der Gleithülse (5) vorspringt, wenn diese sich in der zurückgezogenen Position befindet, wobei die Außenfläche der Nabe (2) die Gleithülse (5) in einer radialen Richtung auslenkt, wenn die Gleithülse (5) in einer axialen Richtung hin zu dem Aufnahmeende der Nabe (2) gleitet, **dadurch gekennzeichnet, dass** in der Nähe des Befestigungsendes ein Federelement (7) an der Gleithülse (5) montiert ist, dass eine Verlagerungskraft die Gleithülse (5) in Richtung auf die zurückgezogene Position drückt und eine Rückstellkraft in dem Federelement (7) bereitgestellt wird, wobei die Rückstellkraft die Gleithülse (5) derart beaufschlagt, dass diese sich axial in Richtung auf das Injektionsende der Nabe (2) und in die ausgefahrene Position bewegt, wenn die Verlagerungskraft aufgehoben wird.

2. Sicherheitsnadel (1) nach Anspruch 1, **dadurch gekennzeichnet, dass** die Gleithülse (5) mindestens einen sich längs erstreckenden freitragenden Arm (15) aufweist.

3. Sicherheitsnadel (1) nach Anspruch 2, **dadurch gekennzeichnet, dass** die Gleithülse (5) 2 bis 6 freitragende Arme (15) aufweist.

4. Sicherheitsnadel (1) nach Anspruch 2, **dadurch gekennzeichnet, dass** mindestens ein freitragender Arm (15) in der Nähe eines Injektionsendes (13) der Gleithülse (5) an einem Ende einer Hülse (12; 12a) frei angelenkt und befestigt ist.

5. Sicherheitsnadel (1) nach Anspruch 1, **dadurch gekennzeichnet, dass** die Gleithülse (5) in einer Zwischenposition zwischen der ausgefahrenen Position und der zurückgezogenen Position verschiebbar gehalten ist, wobei die Gleithülse (5) in der axialen Richtung in Richtung auf die zurückgezogene Position beaufschlagt und die Gleithülse (5) aus der Zwischenposition freigegeben wird.

6. Sicherheitsnadel (1) nach Anspruch 5, **dadurch gekennzeichnet, dass** die Gleithülse (5) mindestens einen sich radial erstreckenden Vorsprung und die Nabe (2) mindestens eine Schrägnut hat, wobei der mindestens eine sich radial erstreckende Vorsprung in der Zwischenposition in der mindestens einen Schrägnut gehalten wird.

7. Sicherheitsnadel (1) nach Anspruch 1, **dadurch gekennzeichnet, dass** sich mindestens ein Teil der Außenfläche der Nabe (2) radial nach ihnen in Richtung auf das Injektionsende derart verjüngt, dass im Benutzungsfall, wenn die Nadel (3) in den Patienten eingeführt wird, die Gleithülse (5) durch die verjüngte Außenfläche der Nabe (2) radial nach außen verschoben wird, wenn sich die Gleithülse (5) axial bewegt, wodurch die Rückstellkraft in dem Federelement (7) erzeugt wird.

8. Sicherheitsnadel (1) nach Anspruch 7, **dadurch gekennzeichnet, dass** die Außenfläche der Nabe (2) zumindest teilweise konisch geformt ist.

9. Sicherheitsnadel (1) nach Anspruch 1, **dadurch gekennzeichnet, dass** eine Fluid-Abgabevorrichtung vorgesehen ist und dass das Aufnahmeende der Nabe (2) an der Fluid-Abgabevorrichtung montiert ist.

10. Sicherheitsnadel (1) nach Anspruch 9, **dadurch gekennzeichnet, dass** die Fluid-Abgabevorrichtung eine Spritze (24) ist.

11. Sicherheitsnadel (1) nach Anspruch 1, **dadurch gekennzeichnet, dass** das Injektionsende der Nabe (2) einen Verriegelungsmechanismus aufweist und die Gleithülse (5) infolge der Aufhebung der Verlagerungskraft durch den Verriegelungsmechanismus in der ausgefahrenen Position gehalten wird.

12. Sicherheitsnadel (1) nach Anspruch 11, **dadurch gekennzeichnet, dass** die Gleithülse (5) mindestens einen sich radial nach innen erstreckenden Vorsprung aufweist und der Verriegelungsmechanismus eine Einkerbung in dem Vorsprung der Nabe (2) an dem freien Ende des Auslegers ist, die den zumindest einen Vorsprung in der ausgefahrenen Position aufnimmt.

13. Sicherheitsnadel (1) nach Anspruch 1, **dadurch gekennzeichnet, dass** das Federelement (7) eine umliegende Feder ist.

14. Sicherheitsnadel (1) nach Anspruch 1, **dadurch gekennzeichnet, dass** das Federelement (7) aus einem Elastomermaterial gebildet ist.

15. Sicherheitsnadel (1) nach Anspruch 1, **dadurch gekennzeichnet, dass** das Federelement (7) aus einem metallischen Material gebildet ist.

16. Sicherheitsnadel (1) nach Anspruch 1, **dadurch gekennzeichnet, dass** das Federelement (7) eine Hülle ist, die die Gleithülse (5) im Wesentlichen verdeckt.

17. Sicherheitsnadel (1) nach Anspruch 1, **dadurch gekennzeichnet, dass** die Gleithülse (5) mindestens einen freitragenden Arm (15) hat, der in eine schraubenförmige Bahn in der Außenfläche der Nabe (2) derart eingreift, dass im Verwendungsfall, wenn die Nadel in einen Patienten eingeführt wird und die Gleithülse (5) an den Patienten stößt, der mindestens eine freitragende Arm durch die schraubenförmige Bahn in Umfangsrichtung verlagert wird.

18. Sicherheitsnadel (1) nach Anspruch 1, **dadurch gekennzeichnet, dass** die Gleithülle (5) eine erste ausgefahrene Position, in der sich die Gleithülle (5) in Richtung auf das Aufnahmeende der Nabe (2) und in die zurückgezogene Position bewegen kann, und eine zweite ausgefahrene Position, in der sich die Gleithülse (5) in einer Verriegelungsposition befindet, aufweist.

## Revendications

1. Aiguille de sécurité (1) pour couvrir de manière automatique une aiguille (3) suivant le dégagement de l'aiguille (3) d'un patient, ladite aiguille de sécurité comprenant une aiguille creuse (3) avec un axe longitudinal et une pointe (4) destinée à être injectée au patient; un moyeu (2) monté à l'aiguille (3) et comportant une surface extérieure, une extrémité de réception distale par rapport à la pointe (4) de l'aiguille (3) et une extrémité proximale par rapport à la pointe (4) de l'aiguille (3) ; un manche glissant (5) comprenant une extrémité de montage distale par rapport à la pointe (4) de l'aiguille (3) et une extrémité d'injection (13) proximale par rapport à la pointe (4) de l'aiguille (3), l'extrémité de montage du manche glissant (5) étant monté de manière glissante par rapport au moyeu (2) entre les extrémités d'injection du moyeu (2), la pointe (4) de l'aiguille (3) étant située à l'intérieur du manche glissant (5) dans une position sortie, la pointe (4) de l'aiguille (3) s'élançant en avant du manche glissant (5) dans une position rentrée, la surface extérieure du moyeu (2) fléchissant le manche glissant (5) dans une direction radiale lors du glissement du manche glissant (5) dans une direction axiale vers l'extrémité de réception du moyeu (2), **caractérisée en ce qu'**un élément de ressort (7) est monté au manche glissant (5) à proximité de l'extrémité de montage et **en ce qu'**est prévue une force de déplacement sollicitant le manche glissant (5) vers la position rentrée en générant une force de rappel dans l'élément de ressort (7), la force de rappel sollicitant le manche glissant (5) de sorte que la manche glissant (5) se meut axialement vers l'extrémité d'injection du moyeu (2) et dans la position sortie lors de la levée de la force de déplacement.

2. L'aiguille de sécurité (1) selon la revendication 1, **caractérisé en ce que** le manche glissant (5) comporte au moins un bras cantilever (15) s'étendant longitudinalement.

3. L'aiguille de sécurité (1) selon la revendication 2, **caractérisé en ce que** le manche glissant (5) comporte un nombre de bras cantilever (15) de 2 à 6.

4. L'aiguille de sécurité (1) selon la revendication 2, **caractérisé en ce qu'**au moins un bras cantilever (15) est pivoté et attaché sur une extrémité et de manière libre à une douille (12 ; 12a) à proximité d'une extrémité d'injection (13) du manche glissant (5).

5. L'aiguille de sécurité (1) selon la revendication 1, **caractérisé en ce que** le manche glissant (5) est retenu de manière détachable dans une position intermédiaire entre la position sortie et la position rentrée, sollicitant le manche glissant (5) dans la direction axiale vers la position rentrée et libérant le manche glissant (5) de la position intermédiaire.

6. L'aiguille de sécurité (1) selon la revendication 5, **caractérisé en ce que** le manche glissant (5) comporte au moins une projecture s'étendant radialement, et le moyeu comporte au moins une rainure inclinée, l'au moins une projecture s'étendant radialement étant retenue dans l'au moins une rainure inclinée, dans la position intermédiaire.

7. L'aiguille de sécurité (1) selon la revendication 1, **caractérisé en ce qu'**au moins une partie de la surface extérieure du moyeu (2) se diminue radialement vers l'intérieur et vers l'extrémité d'injection de sorte que, lors de l'usage, lorsque l'aiguille (3) est insérée dans un patient, le manche glissant (5) se meut axialement en générant la force de rappel dans l'élément de ressort (7).

8. L'aiguille de sécurité (1) selon la revendication 7, **caractérisé en ce que** la surface extérieure du moyeu (2) est conique au moins en partie.

9. L'aiguille de sécurité (1) selon la revendication 1, **caractérisé en ce qu'**est prévu un dispositif d'alimentation en fluide et **en ce que** l'extrémité de réception du moyeu (2) est monté au dispositif d'alimentation en fluide.

10. L'aiguille de sécurité (1) selon la revendication 9, **caractérisé en ce que** le dispositif d'alimentation en fluide et une seringue (24).

11. L'aiguille de sécurité (1) selon la revendication 1, **caractérisé en ce que** l'extrémité d'injection du moyeu (2) comporte un mécanisme de verrouillage, et le manche glissant (5) est retenu dans la position sortie par ledit mécanisme de verrouillage suivant la levée de la force de déplacement.

12. L'aiguille de sécurité (1) selon la revendication 11, **caractérisé en ce que** le manche glissant (5) comporte au moins une projecture s'étendant radialement vers l'intérieur et **en ce que** le mécanisme de verrouillage est une indentation dans la projecture du moyeu (2) sur l'extrémité libre du cantilever qui reçoit l'au moins une projecture dans la position sortie.

13. L'aiguille de sécurité (1) selon la revendication 1, **caractérisé en ce que** l'élément de ressort (7) est un ressort entourant.

14. L'aiguille de sécurité (1) selon la revendication 1, **caractérisé en ce que** l'élément de ressort (7) est fabriqué d'un matériau élastomère.

15. L'aiguille de sécurité (1) selon la revendication 1, **caractérisé en ce que** l'élément de ressort (7) est fabriqué d'un matériau métallique.

16. L'aiguille de sécurité (1) selon la revendication 1, **caractérisé en ce que** l'élément de ressort (7) est une enveloppe recouvrant essentiellement le manche glissant (5).

17. L'aiguille de sécurité (1) selon la revendication 1, **caractérisé en ce que** le manche glissant (5) comporte au moins un bras cantilever (15) intervenant dans un trajet en forme d'hélice dans la surface extérieure du moyeu de sorte que, lors de l'usage, lorsque l'aiguille est insérée dans un patient et le manche glissant (5) bute contre le patient, ledit au moins un bras cantilever (15) est déplacé circonferentiellement par ledit trajet en forme d'hélice.

18. L'aiguille de sécurité (1) selon la revendication 1, **caractérisé en ce que** le manche glissant (5) comporte une première position sortie dans laquelle le manche glissant (5) peut être mu vers l'extrémité de réception du moyeu (2) et dans la position rentrée, et une deuxième position sortie dans laquelle le manche glissant (5) se trouve dans la position verrouillée.
